# EUROPEAN PATENT APPLICATION

(11) **EP 1 623 983 A1**
(43) Date of publication of application: **08.02.2006**
(21) Application number: 04018557.1
(22) Date of filing: 05.08.2004
(51) Int. Cl.: C07D 413/04, C07D 271/06, A61P 3/00, A61P 9/00, A61P 25/00, A61K 31/4245

(54) **Heterocyclic compounds useful as DPP-IV inhibitors**

(71) Applicant: Santhera Pharmaceuticals (Deutschland) Aktiengesellschaft, 69120 Heidelberg (DE)
(72) Inventor: Edwards, Paul John, 69115 Heidelberg (DE); Cerezo-Galvez, Silvia, 42287 Wuppertal (DE); Nordhoff, Sonja, 69198 Schriesheim (DE)
(74) Representative: Grünecker, Kinkeldey, Stockmair & Schwanhäusser Anwaltssozietät

(57) **Abstract**

The invention relates to compounds of formula (I)
Z-C(R¹R²)-C(R³ NH₂)-C(R⁴ R⁵)-A (I),
wherein Z, R¹-R⁵ and A have the meaning as cited in the description and the claims, as examplified by the compound 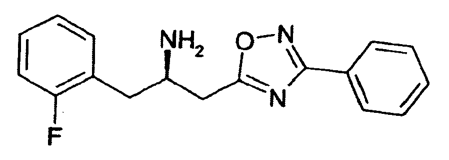 said compounds are useful as DPP-IV inhibitors. The invention also relates to the preparation of such compounds as well as the production and use thereof as medicament.

## Description

The present invention relates to a novel class of dipeptidyl peptidase inhibitors, including pharmaceutically acceptable salts and prodrugs thereof, which are useful as therapeutic compounds, particularly in the treatment of Type 2 diabetes mellitus, often referred to as non-insulin dependent diabetes mellitus (NIDDM), and of conditions that are often associated with this disease, such as obesity and lipid disorders.

Diabetes refers to a disease process derived from multiple causative factors and characterized by elevated levels of plasma glucose or hyperglycemia in the fasting state or after administration of glucose during an oral glucose tolerance test. Persistent or uncontrolled hyperglycemia is associated with increased and premature morbidity and mortality. Often abnormal glucose homeostasis is associated both directly and indirectly with alterations of the lipid, lipoprotein and apolipoprotein metabolism and other metabolic and hemodynamic disease. Therefore patients with Type 2 diabetes mellitus are at an increased risk of macrovascular and microvascular complications, including coronary heart disease, stroke, peripheral vascular disease, hypertension, nephropathy, neuropathy, and retinopathy. Therefore, therapeutic control of glucose homeostasis, lipid metabolism and hypertension are critically important in the clinical management and treatment of diabetes mellitus.

There are two generally recognized forms of diabetes. In Type 1, or insulin-dependent, diabetes mellitus (IDDM), patients produce little or no insulin, which is the hormone regulating glucose utilization. In Type 2, or noninsulin dependent, diabetes mellitus (NIDDM), patients often have plasma insulin levels that are the same or elevated compared to nondiabetic subjects. These patients develop a resistance to the insulin stimulating effect on glucose and lipid metabolism in the main insulin-sensitive tissues, namely the muscle, liver and adipose tissues. Further, the plasma insulin levels, while elevated, are insufficient to overcome the pronounced insulin resistance.

Insulin resistance is not primarily due to a diminished number of insulin receptors but to a post-insulin receptor binding defect that is not yet understood. This resistance to insulin responsiveness results in insufficient insulin activation of glucose uptake, oxidation and storage in muscle, and inadequate insulin repression of lipolysis in adipose tissue and of glucose production and secretion in the liver.

The available treatments for Type 2 diabetes, which have not changed substantially in many years, have recognized limitations. While physical exercise and reductions in dietary intake of calories will dramatically improve the diabetic condition, compliance with this treatment is very poor because of well-entrenched sedentary lifestyles and excess food consumption, especially of foods containing high amounts of saturated fat. Increasing the plasma level of insulin by administration of sulfonylureas (e.g., tolbutamide and glipizide) or meglitinide, which stimulate the pancreatic □-cells to secrete more insulin, and/or by injection of insulin when sulfonylureas or meglitinide become ineffective, can result in insulin concentrations high enough to stimulate the very insulin-resistant tissues. However, dangerously low levels of plasma glucose can result from administration of insulin or insulin secretagogues (sulfonylureas or meglitinide), and an increased level of insulin resistance, due to the even higher plasma insulin levels, can occur. The biguanides increase insulin sensitivity resulting in some correction of hyperglycemia. However, the two biguanides, phenformin and metformin, can induce lactic acidosis and nausea/diarrhoea. Metformin has fewer side effects than phenformin and is often prescribed for the treatment of Type 2 diabetes.

The glitazones (*i*.*e*., 5-benzylthiazolidine-2,4-diones) are a recently described class of compounds with potential for ameliorating many symptoms of Type 2 diabetes. These agents substantially increase insulin sensitivity in muscle, liver and adipose tissue in several animal models of Type 2 diabetes, resulting in partial or complete correction of the elevated plasma levels of glucose without occurrence of hypoglycemia. The glitazones that are currently marketed are agonists of the peroxisome proliferator activated receptor (PPAR), primarily the PPAR-gamma subtype. PPAR-gamma agonism is generally believed to be responsible for the improved insulin sensitization that is observed with the glitazones. Newer PPAR agonists that are being tested for treatment of Type 2 diabetes are agonists of the alpha, gamma or delta subtype, or a combination of these, and in many cases are chemically different from the glitazones (*i*.*e*., they are not thiazolidinediones). Serious side effects (*e.g*., liver toxicity) have occurred with some of the glitazones, such as troglitazone.

Additional methods of treating the disease are still under investigation. New biochemical approaches that have been recently introduced or are still under development include treatment with alpha-glucosidase inhibitors (*e.g*., acarbose) and protein tyrosine phosphatase-1B (PTP-1B) inhibitors.

Compounds that are inhibitors of the dipeptidyl peptidase-IV (DPP-IV) enzyme are also under investigation as drugs that may be useful in the treatment of diabetes, and particularly Type 2 diabetes. See for example WO-A-97/40832, WO-A-98/19998, WO-A-03/180, WO-A-03/181 and WO-A-2004/007468. The usefulness of DPP-IV inhibitors in the treatment of Type 2 diabetes is based on the fact that DPP-IV *in vivo* readily inactivates glucagon like peptide-1 (GLP-1) and gastric inhibitory peptide (GIP). GLP-1 and GIP are incretins and are produced when food is consumed. The incretins stimulate production of insulin. Inhibition of DPP-IV leads to decreased inactivation of the incretins, and this in turn results in increased effectiveness of the incretins in stimulating production of insulin by the pancreas. DPP-IV inhibition therefore results in an increased level of serum insulin. Advantageously, since the incretins are produced by the body only when food is consumed, DPP-IV inhibition is not expected to increase the level of insulin at inappropriate times, such as between meals, which can lead to excessively low blood sugar (hypoglycemia). Inhibition of DPP-IV is therefore expected to increase insulin without increasing the risk of hypoglycemia, which is a dangerous side effect associated with the use of insulin secretagogues.

DPP-IV inhibitors may also have other therapeutic utilities, as discussed elsewhere in this application. DPP-IV inhibitors have not been studied extensively to date, especially for utilities other than diabetes. New compounds are needed so that improved DPP-IV inhibitors can be found for the treatment of diabetes and potentially other diseases and conditions.

Thus, the object of the present invention is to provide a new class of DPP-IV inhibitors which may be effective in the treatment of Type 2 diabetes and other DPP-IV modulated diseases.

Accordingly, the present invention provides novel compounds of formula (I):

Z-C(R¹R²)-C(R³NH₂)-C(R⁴R⁵)-A (I)

or a pharmaceutical acceptable salt thereof, wherein
Z is selected from the group consisting of phenyl; naphthyl; indenyl; C₃₋₇ cycloalkyl; indanyl; tetralinyl; decalinyl; heterocycle; and heterobicycle, wherein Z is optionally substituted with one or more R⁶, wherein R⁶ is independently selected from the group consisting of halogen; CN; OH; NH₂; oxo (=O), where the ring is at least partially saturated; R⁷; and R⁸;
R⁷ is selected from the group consisting of C₁₋₆ alkyl; O-C₁₋₆ alkyl; and S-C₁₋₆ alkyl, wherein R⁷ is optionally interrupted by oxygen and wherein R⁷ is optionally substituted with one or more halogen independently selected from the group consisting of F; and Cl;
R⁸ is selected from the group consisting of phenyl; heterocycle; and C₃₋₇ cycloalkyl, wherein R⁸ is optionally substituted with one or more R⁹, wherein R⁹ is independently selected from the group consisting of halogen; CN; OH; NH₂; oxo (=O), where the ring is at least partially saturated; C₁₋₆ alkyl; O-C₁₋₆ alkyl; and S-C₁₋₆ alkyl;
R¹, R⁴ are independently selected from the group consisting of H; F; OH; and R¹⁰;
R², R⁵ are independently selected from the group consisting of H; F; and R¹¹;
R¹⁰ is independently selected from the group consisting of C₁₋₆ alkyl; O-C₁₋₆ alkyl; N(R¹²)-C₁₋₆ alkyl; S-C₁₋₆ alkyl; C₃₋₇ cycloalkyl; O-C₃₋₇ cycloalkyl; N(R¹²)-C₃₋₇ cycloalkyl;
S-C₃₋₇ cycloalkyl; -C₁₋₆ alkyl-C₃₋₇ cycloalkyl; O-C₁₋₆ alkyl-C₃₋₇ cycloalkyl; N(R¹²)-C₁₋₆ alkyl-C₃₋₇ cycloalkyl; S-C₁₋₆ alkyl-C₃₋₇ cycloalkyl; heterocycle; O-heterocycle; N(R¹²)-heterocycle; S-heterocycle; C₁₋₆ alkyl-heterocycle; O-C₁₋₆ alkyl-heterocycle; N(R¹²)-C₁₋₆ alkyl-heterocycle; S-C₁₋₆ alkyl-heterocycle; wherein R¹⁰ is optionally substituted with one or more halogen independently selected from the group consisting of F; and Cl;
R¹² is selected from the group consisting of H; and C₁₋₆ alkyl;
R¹¹ is independently selected from the group consisting of C₁₋₆ alkyl; C₃₋₇ cycloalkyl; and -C₁₋₆ alkyl-C₃₋₇ cycloalkyl, wherein R¹¹ is optionally substituted with one or more R¹³, wherein R¹³ is independently selected from the group consisting of F; Cl; and OH;
R³ is selected from the group consisting of H; and C₁₋₆ alkyl;

Optionally one or more pairs of R¹, R², R³, R⁴, R⁵ independently selected from the group consisting of R¹/R²; R²/R³ and R⁴/R⁵; form a C₃₋₇ cycloalkyl ring, which is optionally substituted with one or more of R¹⁴, wherein R¹⁴ is independently selected from the group consisting of F; Cl; and OH;

A is selected from the group consisting of a 5-membered heterocycle having two double bonds and with at least one nitrogen and one additional heteroatom selected from the group consisting of nitrogen; oxygen; and sulphur, as ring atoms; and a 6-membered heterocycle having three double bonds and with at least one nitrogen and one additional heteroatom selected from the group consisting of nitrogen; oxygen; and sulphur, as ring atoms; wherein A is substituted with one or more R¹⁵;

Optionally, A is substituted with one or more R¹⁶, wherein R¹⁶ is independently selected from the group consisting of F; OH; O-C₁₋₆ alkyl; NH₂; NH-C₁₋₆ alkyl; N(C₁₋₆ alkyl)₂; SH; S-C₁₋₆ alkyl; and C₁₋₆ alkyl, wherein each C₁₋₆ alkyl is optionally substituted with one or more halogen selected from the group consisting of F; and Cl;
R¹⁵ is selected from the group consisting of T; -Y-H; and -Y-T;
Y is selected from the group consisting of a covalent bond; -C₁₋₆ alkyl-T⁰-; -O-T⁰-; -O-T⁰-C₁₋₆ alkyl-; -C₁₋₆ alkyl-O-T⁰-; -S-T⁰-; -S-T⁰-C₁₋₆ alkyl-; -C₁₋₆ alkyl-S-T⁰-; -S(O)-T⁰-;
-S(O) -T⁰-C₁₋₆ alkyl-; -S(O)₂-T⁰-; -S(O)₂-T⁰-C₁₋₆ alkyl-; -C₁₋₆ alkyl-S(O)-T⁰-; -C₁₋₆ alkyl-S(O)₂-T⁰-; -N(R¹⁷)-T⁰-; -N(R¹⁷)-T⁰-C₁₋₆ alkyl-; -C₁₋₆ alkyl-N(R¹⁷)-T⁰-; -C(O)-O-;
-C(O)O-C₁₋₆ alkyl-T⁰-; -C₁₋₆ alkyl-C(O)O-; -C₁₋₆ alkyl-C(O)O-C₁₋₆ alkyl-T⁰-;-C(O)N(R¹⁷)-; -C(O)N(R¹⁷)-C₁₋₆ alkyl-T⁰-; -C₁₋₆ alkyl-C(O)N(R¹⁷)-; and -C₁₋₆ alkyl-C(O)N(R¹⁷)- C₁₋₆ alkyl-T⁰-; wherein each C₁₋₆ alkyl is optionally substituted with one or more F;
T⁰ is selected from the group consisting of a covalent bond; -C₁₋₆ alkyl-; -C₁₋₆ alkyl-O-; -C₁₋₆ alkyl-N(R¹⁸)-; -C(O)-; -C(O)-C₁₋₆ alkyl-; -C(O)-C₁₋₆ alkyl-O-; -C(O)-C₁₋₆ alkyl-N(R¹⁸)-;
-C(O)O-; -C(O)O-C₁₋₆ alkyl-; -C(O)O-C₁₋₆ alkyl-O-; -C(O)O-C₁₋₆ alkyl-N(R¹⁸)-;
-C(O)N(R¹⁸)-; -C(O)N(R¹⁸)-C₁₋₆ alkyl-; -C(O)N(R¹⁸)-C₁₋₆ alkyl-O-;
-C(O)N(R¹⁸)-C₁₋₆ alkyl-N(R¹⁹)-; -S(O)₂-N(R¹⁸)-; -S(O)₂-N(R¹⁸)-C₁₋₆ alkyl-; -S(O)₂₋N(R¹⁸)-C₁₋₆ alkyl-O-; -S(O)₂N(R¹⁸)-C₁₋₆ alkyl-N(R¹⁹)-; -S(O)₂-; -S(O)₂-C₁₋₆ alkyl-; - S(O)₂-C₁₋₆ alkyl-O-; and -S(O)₂-C₁₋₆ alkyl-N(R¹⁸)-; wherein each C₁₋₆ alkyl is optionally substituted with one or more F;
R¹⁷, R¹⁸, R¹⁹ are independently selected from the group consisting of H; and C₁₋₆ alkyl;
T is selected from the group consisting of T¹; and T²;
T¹ is selected from the group consisting of phenyl; naphthyl; and indenyl; wherein T¹ is optionally substituted with one or more R²⁰; wherein R²⁰ is independently selected from the group consisting of halogen; CN; R²¹; COOH; OH; C(O)NH₂; S(O)₂NH₂; S(O)NH₂; COOT³; OT³; ST³; C(O)N(R²²)T³; N(R²²)S(O)T³; N(R²²)S(O)₂T³; S(O)₂N(R²²)T³; S(O)N(R²²)T³ and T³;
T² is selected from the group consisting of C₃₋₇ cycloalkyl; indanyl; tetralinyl; decalinyl; heterocycle; and heterobicycle; wherein T² is optionally substituted with one or more R²³, wherein R²³ is independently selected from the group consisting of halogen; CN; R²⁴; OH; oxo (=O), where the ring is at least partially saturated; NH₂; COOH; C(O)NH₂; S(O)₂NH₂; S(O)NH₂; COOT³; OT³; C(O)N(R²²)T³; N(R²²)S(O)T³; N(R²²)S(O)₂T³; S(O)₂N(R²²)T³; S(O)N(R²²)T³; N(R²²)T³; and T³;

Optionally R²³ is C(O)R²², provided that C(O)R²² is bound to a nitrogen, which is a ring atom of a heterocycle or heterobicycle;
R²¹ is selected from the group consisting of C₁₋₆ alkyl; O-C₁₋₆ alkyl; S-C₁₋₆ alkyl; COO-C₁₋₆ alkyl; OC(O)- C₁₋₆ alkyl; C(O)N(R²⁵)-C₁₋₆ alkyl; S(O)₂N(R²⁵)-C₁₋₆ alkyl; S(O)N(R²⁵)-C₁₋₆ alkyl; S(O)-C₁₋₆ alkyl; S(O)₂-C₁₋₆ alkyl; N(R²⁵)S(O)₂-C₁₋₆ alkyl; and N(R²⁵)S(O) -C₁₋₆ alkyl; wherein each C₁₋₆ alkyl is optionally substituted with one more R²⁶, wherein R²⁶ is independently selected from the group consisting of F; COOR²⁷; C(O)N(R²⁷R²⁸); S(O)₂N(R²⁷R²⁸); OR²⁷; N(R²⁷R²⁸); T³; O-T³; and N(R²⁷)-T³;
R²⁴ is selected from the group consisting of C₁₋₆ alkyl; O-C₁₋₆ alkyl; S-C₁₋₆ alkyl; N(R²⁵)-C₁₋₆ alkyl; COO-C₁₋₆ alkyl; OC(O)- C₁₋₆ alkyl; C(O)N(R²⁵)- C₁₋₆ alkyl; N(R²⁵)-C(O)-C₁₋₆ alkyl; S(O)₂N(R²⁵)-C₁₋₆ alkyl; S(O)N(R²⁵)-C₁₋₆ alkyl; S(O)-C₁₋₆ alkyl; S(O)₂-C₁₋₆ alkyl; -N(R²⁵)S(O)₂-C₁₋₆ alkyl; and -N(R²⁵)S(O)-C₁₋₆ alkyl; wherein each C₁₋₆ alkyl is optionally substituted with one or more R^{26a}, wherein R^{26a} is independently selected from the group consisting of F; COOR²⁷; C(O)N(R²⁷R²⁸); S(O)₂N(R²⁷R²⁸); S(O)N(R²⁷R²⁸); OR²⁷; N(R²⁷R²⁸); T³; O-T³; and N(R²⁷)-T³;
R²², R²⁵, R²⁷, R²⁸ are independently selected from the group consisting of H; and C₁₋₆ alkyl;
T³ is selected from the group consisting of T⁴; and T⁵;
T⁴ is selected from the group consisting of phenyl; naphthyl; and indenyl; wherein T⁴ is optionally substituted with one or more R²⁹, wherein R²⁹ is independently selected from the group consisting of halogen; CN; COOR³⁰; OR³⁰; C(O)N(R³⁰R³¹); S(O)₂N(R³⁰R³¹); C₁₋₆ alkyl; O-C₁₋₆ alkyl; S-C₁₋₆ alkyl; COO-C₁₋₆ alkyl; OC(O)-C₁₋₆ alkyl; C(O)N(R³⁰)-C₁₋₆ alkyl; S(O)₂N(R³⁰)-C₁₋₆ alkyl; S(O)N(R³⁰)-C₁₋₆ alkyl; S(O)₂-C₁₋₆ alkyl; S(O)-C₁₋₆ alkyl; N(R³⁰)S(O)₂-C₁₋₆ alkyl; and N(R³⁰)S(O)-C₁₋₆ alkyl; wherein each C₁₋₆ alkyl is optionally substituted with one more halogen selected from the group consisting of F; and Cl;
T⁵ is selected from the group consisting of heterocycle; heterobicycle;
C₃₋₇ cycloalkyl; indanyl; tetralinyl; and decalinyl; wherein T⁵ is optionally substituted with one or more R³², wherein R³² is independently selected from the group consisting of halogen; CN; OR³⁰; oxo (=O), where the ring is at least partially saturated;
N(R³⁰R³¹); COOR³⁰; C(O)N(R³⁰R³¹); S(O)₂N(R³⁰R³¹); S(O)N(R³⁰R³¹); C₁₋₆ alkyl; O-C₁₋₆ alkyl; S-C₁₋₆ alkyl; N(R³⁰)-C₁₋₆ alkyl; COO-C₁₋₆ alkyl; OC(O)-C₁₋₆ alkyl; C(O)N(R³⁰)-C₁₋₆ alkyl;
N(R³⁰)-C(O)-C₁₋₆ alkyl; S(O)₂N(R³⁰)-C₁₋₆ alkyl; S(O)N(R³⁰)-C₁₋₆ alkyl; S(O)₂-C₁₋₆ alkyl;
S(O)-C₁₋₆ alkyl; N(R³⁰)S(O)₂-C₁₋₆ alkyl; and N(R³⁰)S(O)-C₁₋₆ alkyl; wherein each C₁₋₆ alkyl is optionally substituted with one more halogen selected from the group consisting of F; and Cl;

Optionally R³² is C(O)R³⁰, provided that C(O)R³⁰ is bound to a nitrogen, which is a ring atom of a heterocycle or heterobicycle;
R³⁰, R³¹ are independently selected from the group consisting of H; C₁₋₆ alkyl; C₃₋₇ cycloalkyl; and -C₁₋₆ alkyl-C₃₋₇ cycloalkyl.

Within the meaning of the present invention the terms are used as follows:

In case a variable or substituent can be selected from a group of different variants and such variable or substituent occurs more than once the respective variants can be the same or different.

"Alkyl" means a straight-chain or branched carbon chain that may contain double or triple bonds. It is generally preferred that alkyl doesn't contain double or triple bonds. "C₁₋₄ Alkyl" means an alkyl chain having 1 - 4 carbon atoms, e.g. at the end of a molecule methyl, ethyl, -CH=CH₂, -C≡CH, n-propyl, isopropyl, -CH=CH-CH₃, -CH₂₋CH=CH₂, n-butyl, isobutyl, -CH=CH-CH₂-CH₃, -CH=CH-CH=CH₂, sec-butyl tert-butyl or amid, e.g. -CH₂-, -CH₂-CH₂-, -CH=CH-, -CH(CH₃)-, -C(CH₂)-, -CH₂-CH₂-CH₂-, - CH(C₂H₅)-, -CH(CH₃)₂-.
"C₁₋₆ Alkyl" means an alkyl chain having 1 - 6 carbon atoms, e.g. C₁₋₄ alkyl, methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl, n-pentane, n-hexane, or amid, e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-. Each hydrogen of a C₁₋₆ alkyl carbon may be replaced by a substituent.

"C₃₋₇ Cycloalkyl" or "C₃₋₇ Cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Each hydrogen of a cycloalkyl carbon may be replaced by a substituent.

"Halogen" means fluoro, chloro, bromo or iodo. It is generally preferred that halogen is fluoro or chloro.

"Heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine. "Heterocycle" means also azetidine.

"Heterobicycle" means a heterocycle which is condensed with phenyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, dihydroquinoline, isoquinoline, tetrahydroisoquinoline, dihydroisoquinoline, benzazepine, purine or pteridine.

A preferred stereochemistry of compounds or a pharmaceutically acceptable salt thereof according to the present invention is shown in formula (Ia): 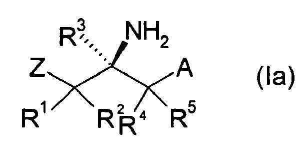
or a pharmaceutically acceptable salt thereof, wherein Z, R¹-R⁵ and A have the meaning as indicated above.

Preferred compounds of formula (I) or (Ia) are those compounds in which one or more of the residues contained therein have the meanings given below, with all combinations of preferred substituent definitions being a subject of the present invention. With respect to all preferred compounds of the formulas (I) or (Ia) the present invention also includes all tautomeric and stereoisomeric forms and mixtures thereof in all ratios, and their pharmaceutically acceptable salts.

In preferred embodiments of the present invention, the Z, R¹-R⁵ and A of the formula (I) or (Ia) independently have the following meaning. Hence, one or more of the substituents Z, R¹-R⁵ and A can have the preferred or more preferred meanings given below.

Preferably, Z is selected from the group consisting of phenyl; and heterocycle; and wherein Z is optionally substituted with up to three R⁶, which are the same or different.

Preferably, R⁶ is selected from the group consisting of F; Cl; CN; and C₁₋₆ alkyl.

Preferably, R¹, R², R⁴, R⁵ are independently selected from the group consisting of H; F; and C₁₋₆ alkyl, optionally substituted with one or more F.

R³ is preferably H.

Preferably, A is selected from the group consisting of oxadiazole; thiadiazole; and triazole.

Preferably, A is substituted with one R¹⁵.

Preferably, Y is a covalent bond.

Preferably, R¹⁵ is phenyl, optionally substituted with up to three R²⁰, which are the same or different.

Preferably, R²⁰ is selected from the group consisting of CH₃; CH₂F; CHF₂; CF₃; CH₂CF₃; F; Cl; S(O)₂NH₂; and CH₃S(O)₂.

Preferably, R¹⁵ is a heterocycle, more preferred pyridine.

Compounds of the formula (I) or (Ia) in which some or all of the above-mentioned groups have the preferred or more preferred meanings are also an object of the present invention.

Preferred embodiments of the compounds according to present invention are:

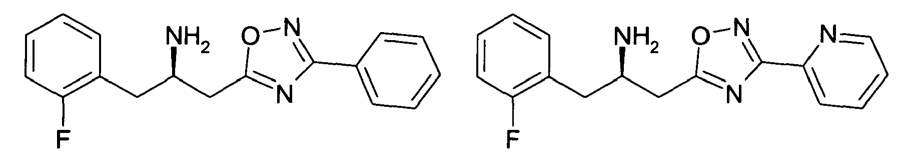

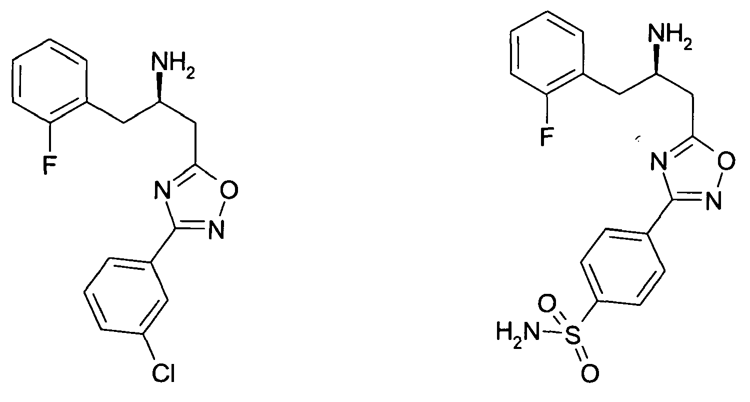

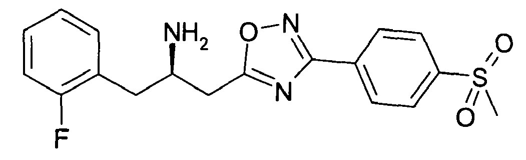

Furthermore, the present invention provides prodrug compounds of the compounds of the invention as described above.
"Prodrug compound" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

Metabolites of compounds of formula (I) or (Ia) are also within the scope of the present invention.

Where tautomerism, like e.g. keto-enol tautomerism, of compounds of general formula (I) or (Ia) or their prodrugs may occur, the individual forms, like e.g. the keto and enol form, are claimed separately and together as mixtures in any ratio. Same applies for stereoisomers, like e.g. enantiomers, cis/trans isomers, conformers and the like.
If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) or (Ia) may be obtained from stereoselective synthesis using optically pure starting materials.

In case the compounds according to formula (I) or (Ia) contain one or more acidic or basic groups, the invention also comprises their corresponding pharmaceutically or toxicologically acceptable salts, in particular their pharmaceutically utilizable salts. Thus, the compounds of the formula (I) or (Ia) which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) or (Ia) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) or (Ia) simultaneously contain acidic and basic groups in the molecule, the invention also includes, in addition to the salt forms mentioned, inner salts or betaines (zwitterions). The respective salts according to the formula (I) or (Ia) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) or (Ia) which, owing to low physiological compatibility, are not directly suitable for use in pharmaceuticals but which can be used, for example, as intermediates for chemical reactions or for the preparation of pharmaceutically acceptable salts.

The present invention provides compounds of general formula (I) or (Ia) or their prodrugs as DPP-IV inhibitors. DPP-IV is a cell surface protein that has been implicated in a wide range of biological functions. It has a broad tissue distribution (intestine, kidney, liver, pancreas, placenta, thymus, spleen, epithelial cells, vascular endothelium, lymphoid and myeloid cells, serum), and distinct tissue and cell-type expression levels. DPP-IV is identical to the T cell activation marker CD26, and it can cleave a number of immunoregulatory, endocrine, and neurological peptides *in vitro.* This has suggested a potential role for this peptidase in a variety of disease processes.

DPP-IV related diseases are described in more detail in WO-A-03/181 under the paragraph "Utilides" which is herewith incorporated by reference.

Accordingly, the present invention provides compounds of formula (I) or (Ia) or their prodrugs or pharmaceutically acceptable salt thereof for use as a medicament.

Furthermore, the present invention provides the use of compounds of formula (I) or (Ia) or their prodrugs or a pharmaceutically acceptable salt thereof for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; retinopathy; nephropathy; neuropathy; Syndrome X; ovarian hyperandrogenism (polycystic ovarian syndrome; Type n diabetes; or growth hormone deficiency. Preferred is non-insulin dependent (Type II) diabetes mellitus and obesity.

The present invention provides pharmaceutical compositions comprising a compound of formula (I) or (Ia), or a prodrug compound thereof, or a pharmaceutically acceptable salt thereof as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

A pharmaceutical composition of the present invention may additionally comprise one or more other compounds as active ingredients like one or more additional compounds of formula (I) or (Ia), or a prodrug compound or other DPP-IV inhibitors.
Other active ingredients are disclosed in WO-A-03/181 under the paragraph "Combination Therapy" which is herewith incorporated by reference.
Accordingly, other active ingredients may be insulin sensitizers; PPAR agonists; biguanides; protein tyrosinephosphatase-1B (PTP-1B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoIy dual agonists; inhibitors of cholesterol absorption; acyl CoA : cholesterol acyltransferase inhibitors; anti-oxidants; PPARo agonists; antiobesity compounds; an ileal bile acid transporter inhibitor; or anti-inflammatory agents or pharmaceutically acceptable salts of these active compounds.

The term "pharmaceutically acceptable salts" refers to salts prepared from pharmaceutically acceptable non-toxic bases or acids, including inorganic bases or acids and organic bases or acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of formula (I) or (Ia) can be combined as the active ingredient in intimate admixture with a pharmaceutical carrier according to conventional pharmaceutical compounding techniques. The carrier may take a wide variety of forms depending on the form of preparation desired for administration, *e.g*., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I) or (Ia) may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (*e.g*., glycerol, propylene glycol and liquid polyethylene glycol), suitable mixtures thereof, and vegetable oils.
Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I) or (Ia) are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing diabetes mellitus and/or hyperglycemia or hypertriglyceridemia or other diseases for which compounds of Formula I are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligram to about 50 milligrams. In the case of a 70 kg adult human, the total daily dose will generally be from about 7 milligrams to about 350 milligrams. This dosage regimen may be adjusted to provide the optimal therapeutic response.

Preferred embodiments of compounds having formula (I) of the present invention can be prepared from nitrile intermediates such as those of formula (III), by coupling with suitably protected beta amino acids having the formula (II) using standard coupling conditions, followed by intramolecular cyclisation. The preparation of these intermediates is described in the following schemes.

Some abbreviations that may appear in this application are as follows.

### ABBREVIATIONS

| Designation | |
|---|---|
| bs | Broad singlet |
| Boc (or BOC) | *tert*-Butoxycarbonyl |
| CDI | *N,N-*Carbonyldiimidazole |
| DCM | Dichloromethane |
| DIC | 1,3-Di-*iso*-propylcarbodiimide |
| HCl | Hydrogen chloride |
| HPLC | High pressure liquid chromatography |
| M.P. | Melting point |
| NMR | Nuclear Magnetic Resonance |
| PG | Protecting group |
| rt | Retention time |
| tert | Tertiary-butyl |
| TFA | Trifluoroacetic acid |
| TLC | Thin Layer Chromatography |

Available starting materials may be enantiomerically pure beta amino acids having the formula (II) or nitriles having the formula (III).

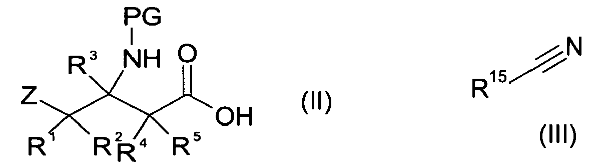

They may be purchased from commercially available sources such as Astatech, Sigma-Aldrich, or be synthesised by one skilled in the art. The conversion of diverse functional groups (such as esters, alcohols, amides, nitriles, azides) may allow the synthesis of some intermediates or final compounds. Enantiomerically pure beta amino acids having the formula (II) where R¹ - R⁵ is H may be commercially available, known in the literature or may be conveniently synthesised using one of the methods already published and reviewed in e.g., Cole, *Tetrahedron,* 32, 9517 (1994), Juaristi et al., *Aldrichimica Acta,* 27, 3, 1994, or Juaristi, *Enantioselective Synthesis of □-Amino Acids*, Ed. Wiley-VCH, New York, 1997.

Schemes A and E outline general procedures for the synthesis of some compounds described below. Unless otherwise indicated in the schemes, the variables have the same meaning as described above.

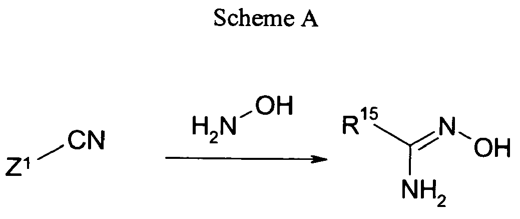

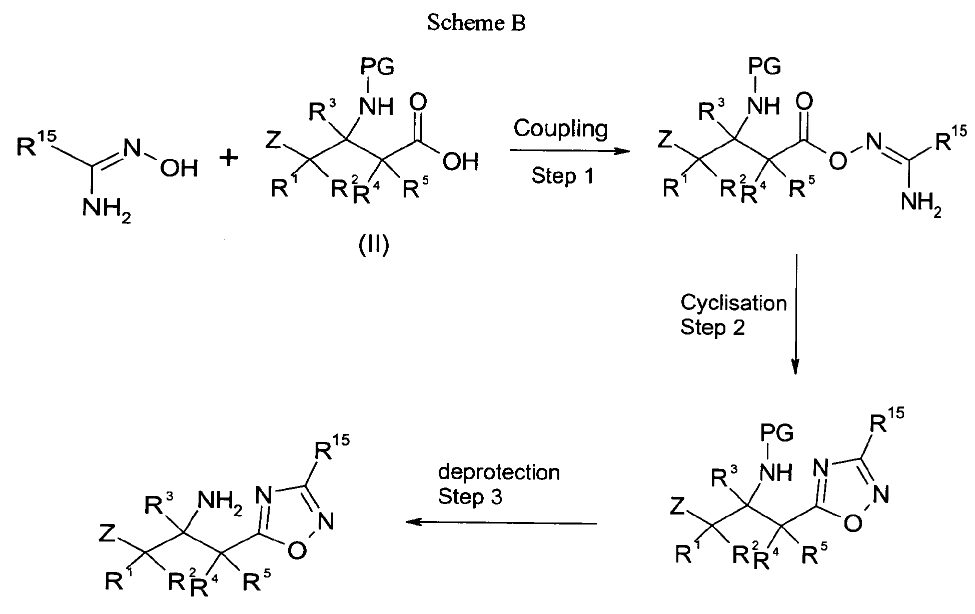

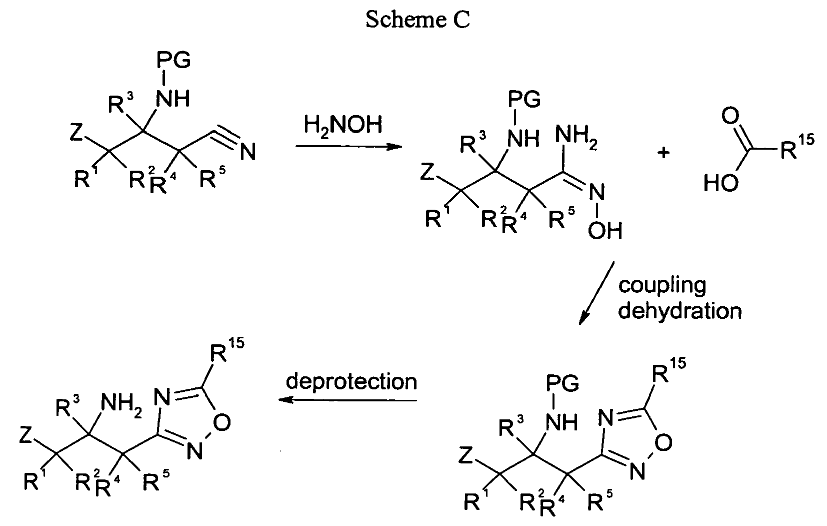

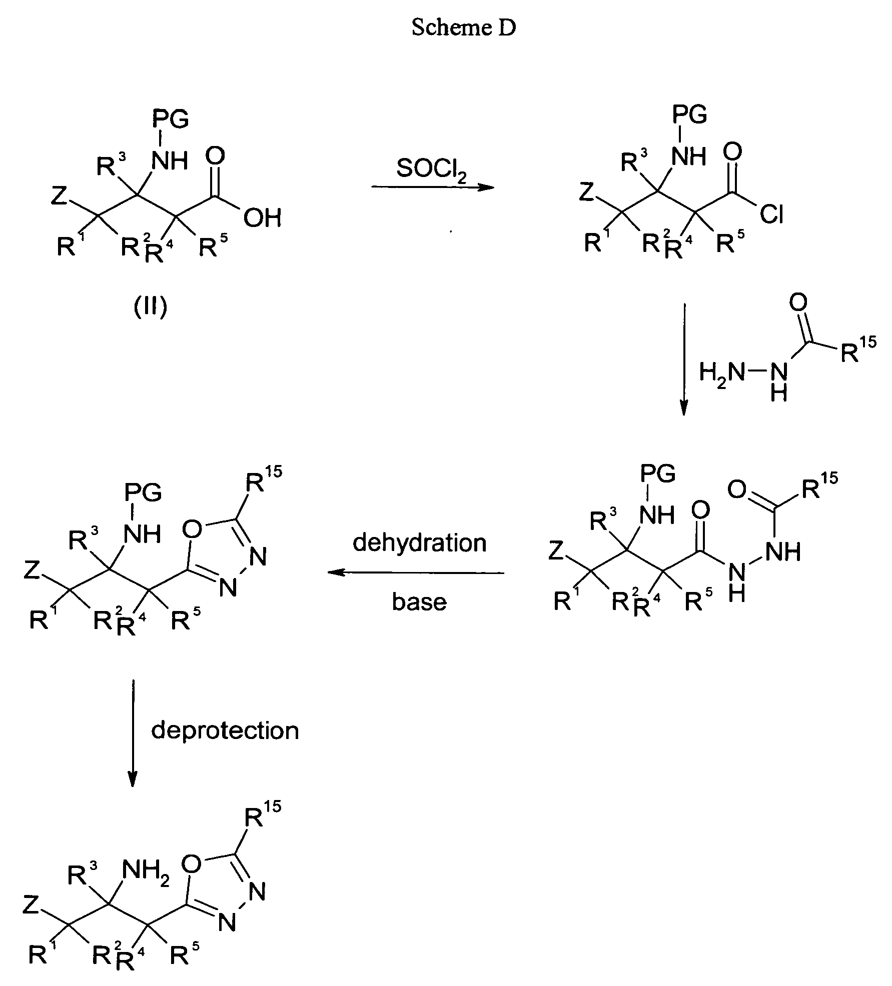

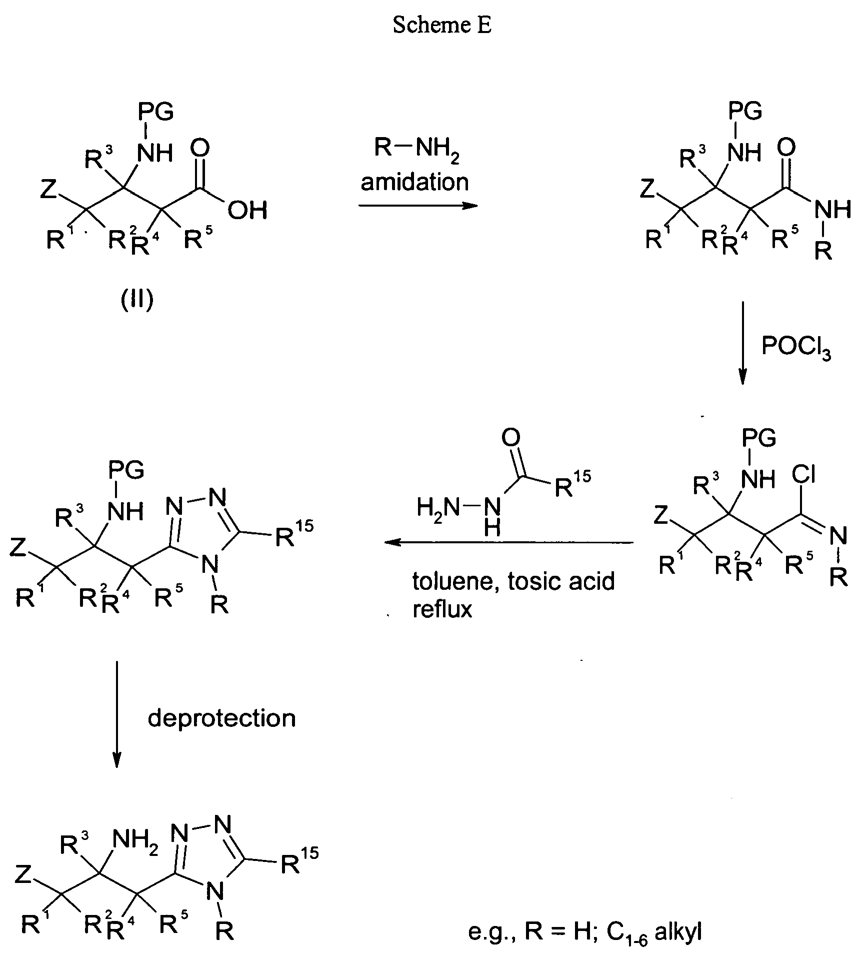

Unless otherwise noted, all non-aqueous reactions were carried out either under an argon or nitrogen atmosphere with commercial dry solvents. Compounds were purified using flash column chromatography using Merck silica gel 60 (230-400 mesh), or by preparative thin layer chromatography using Kieselgel Merck 5554 sheets, or reverse phase preparative HPLC using a Reprosil-Pur ODS3, 5 µm, 20 x 125 mm column with Shimadzu LC8A-Pump and SPD-10Avp UV/Vis diode array detector. The ¹H-NMR spectra were recorded on a Varian VXR-S (300 MHz for ¹H-NMR) using d₆₋dimethylsulfoxide as solvent; chemical shifts are reported in ppm relative to tetramethylsilane. Analytical LC/MS was performed using Reprosil-Pur ODS3, 5 µM, 1 x 60 mm columns with a linear gradient from 5% to 95% acetonitrile in water (0.1% TFA) at a flow rate of 250 µl/min; retention times are given in minutes. Methods are: (I) runs on a LC10Advp-Pump (Shimadzu) with SPD-M10Avp UV/Vis diode array detector and QP2010 MS-detector in ESI+ modus with UV-detection at 214, 254 and 275 nm, 10 min. linear gradient; (II) idem but 5 min. linear gradient; (III) runs on a LC10Advp-Pump (Shimadzu) with SPD-10Avp dual wavelength UV-detector and QP2010 MS-detector in ESI+ modus with UV-detection at 214 and 254 nm, 10 min. linear gradient; (IV) idem but 5 min. linear gradient.

### General procedure for making compounds of the invention

In general, compounds having the structure (I)

Z-C(R¹R²)-C(R³NH₂)-C(R⁴R⁵)-A (I)

wherein the variables have the above described meanings, may be prepared using standard peptide coupling conditions. For example, it may be possible to use 1-ethyl-3-(3-dimethylaminopropyl)carbodiimide hydrochloride (EDC) in combination with 1-hydroxybenzotriazole (HOBt) and a base (triethylamine or diisopropylethylamine) or O-(7-azabenzotriazol-1-yl)-*N,N,N',N'*-tetramethyluronium hexafluorophosphate (HATU) in the presence of a base, in solvents such as methylene chloride or *N,N-*dimethylformamide.

Scheme F outlines a procedure for using the nitriles of formula (III) to synthesize compounds that are embodiments of the invention.

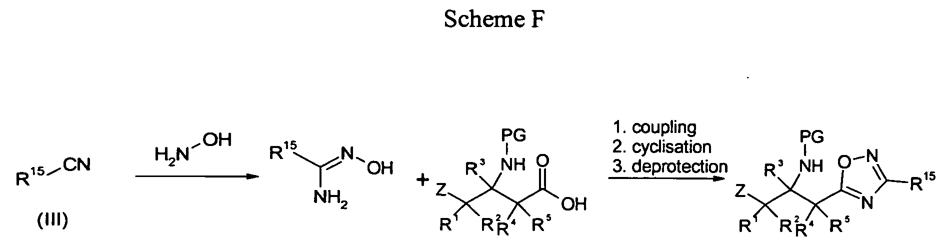

The protective group may be removed with, for example, diethylamine in dichloromethane in the case of 9-fluorenylmethoxycarbonyl or using acidic conditions (such as trifluoroacetic acid in dichloromethane or hydrochloric acid in dioxane) in the case of *tert*-butoxycarbonyl, as described in *Protective Groups in Organic Synthesis* 3^{rd} ed., Ed. Wiley-VCH, New York; 1999.

For the purification of intermediates or end products, flash chromatography on silica gel may be suitable for the free amines whereas the use of preparative HPLC leads to the isolation of the corresponding trifluoroacetic acid salts.

Compounds may be prepared by other means however, and the suggested starting materials and procedures described below are exemplary only and should not be considered as limiting the scope of the invention.

### EXAMPLES

The following examples are provided so that the invention might be more fully understood. These examples are illustrative only and should not be construed as limiting the invention in any way.

### PREPARATIONS

### Example 1

Procedure for making an intermediate according to Scheme A.

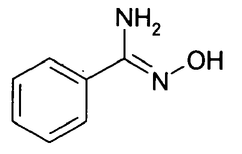

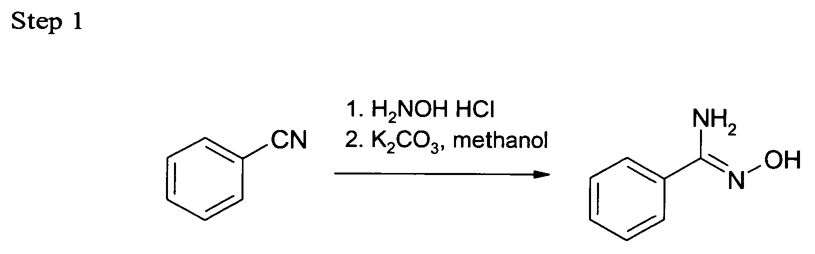

### N-Hydroxybenzamidine

To 10.31 g (0.10 mol) of benzonitrile dissolved in 40 mL methanol is added 20.73 g (0.15 mole) of finely powdered potassium carbonate. To this is added, in small portions with stirring, 13.89 g (0.20 mol) of hydroxylamine hydrochloride dissolved in 120 mL of methanol. The mixture is then refluxed for 5 hours and, after cooling to ambient temperature, the solvent is removed under reduced pressure. The residue is taken up in 50 mL of water and 200 mL of chloroform. The organic layer is separated, washed twice with 30 mL of water, and dried over magnesium sulphate. The mixture is then filtered and evaporated in vacuum. The residue is crystallised with diethyl ether to afford the title compound.
M.P.: 77-79 °C.

### Example 2

Prepared following the procedure outlined for Example 1 according to Scheme A.

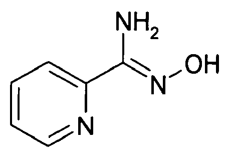

### N-Hydroxypyridine-2-carboxamide

Obtained from pyridine-2-carbonitrile and hydroxylamine hydrochloride according to Step 1 in Example 1.
M.P.: 115-117 °C

### Example 3

Prepared following the procedure outlined for Example 1 according to Scheme A.

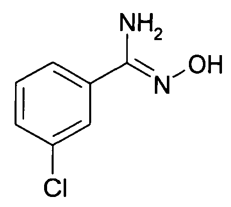

### 3-Chloro-N-hydroxy-benzamidine

Obtained from 3-chlorobenzonitrile and hydroxylamine hydrochloride according to Step 1 in Example 1.
M.P.: 115-118°C

### Example 4

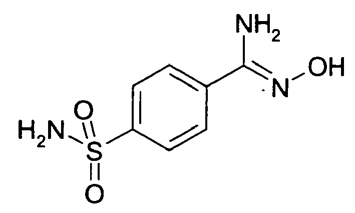

### N-Hydroxy-4-sulphamoyl-benzamidine

To a mixture of 364.90 mg (2.00 mmol) 4-cyanobenzenesulphonamide and 690.30 mg (4.99 mmol) potassium carbonate in 4 mL ethanol is added 166.60 mg (2.40 mmol) of hydroxylamine hydrochloride. The reaction mixture is then refluxed overnight. After cooling to ambient temperature, the white solid is filtered of, washed sequentially with 5mL ethanol and 5 mL methanol. The organic layer is evaporated under reduced pressure to yield the title compound which is taken directly into the next step without further purification.
LC/MS (Method II) rt 0.68 minutes, m/z 216 [M+H]⁺

### Example 5

Prepared following the procedure outlined for Example 1 according to Scheme A.

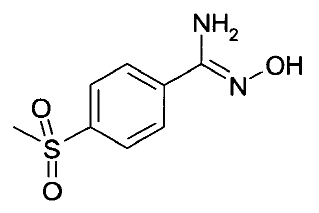

### N-Hydroxy-4-methanesulphonyl-benzamidine

Obtained from 4-methanesulphonyl-benzonitrile and hydroxylamine hydrochloride according to Step 1 in Example 1.
M.P.: 115-118°C

### Example 6

Following examples are prepared according to Schemes B and F.

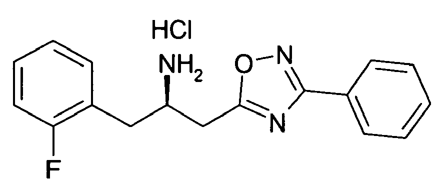

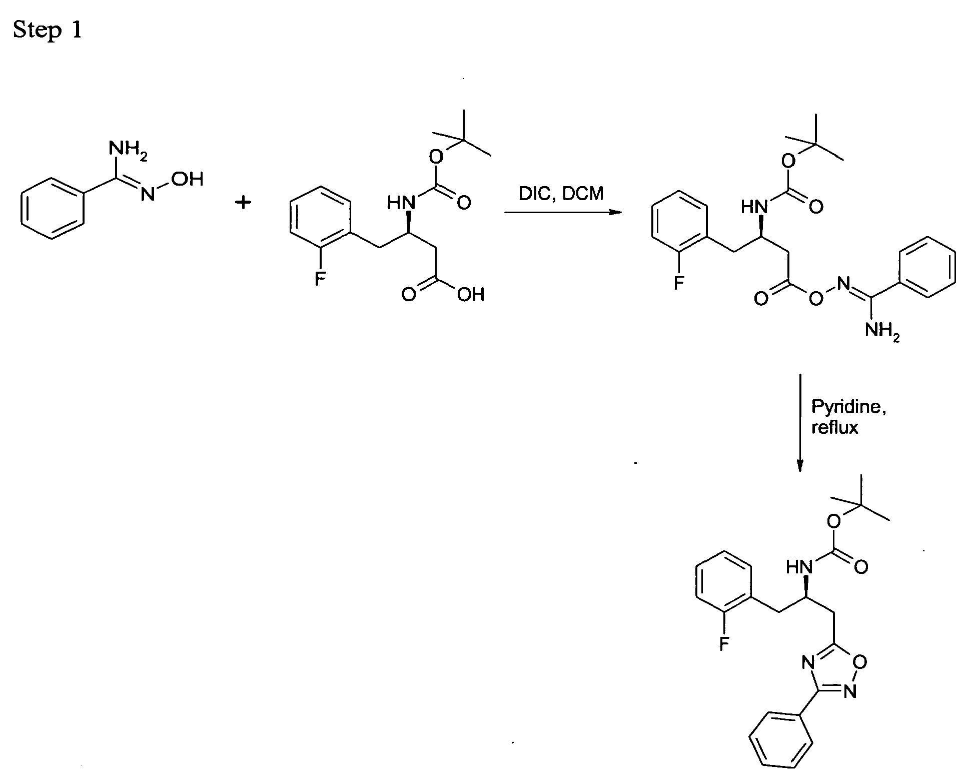

### [2-(2-Fluorophenyl)-(1R)-(3-phenyl-[1,2,4]oxadiazol-5-ylmethyl)-ethyl]-carbamic acid tert-butyl ester

To a mixture of 33.4 mg (0.25 mmol) of *N*-hydroxybenzamidine (Example 1) and 80.0 mg (0.27 mmol; 1.1 eq.) of (3*R*)-*tert*-butoxycarbonylamino-4-(2-fluoro-phenyl)-butyric acid in 5 mL of dry dichloromethane is added 43.6 mg (0.27 mmol) of 1,3-di-*iso*-propylcarbodiimide. The mixture is stirred for 16-18 hours at room temperature under nitrogen. The progress of the reaction is monitored by TLC (Kieselgel Merck 5554 sheets, eluent: dichloroethane-ethanol 5:1). When the formation of the opened-chain intermediate is complete, the reaction mixture is evaporated to dryness. Then the residue is dissolved in 5 mL of dry pyridine and refluxed for 8-10 hours (monitored by TLC: Kieselgel Merck 5554 sheets, eluent: dichloroethane-ethanol 5:1) until the ring-closure reaction is complete. The pyridine is evaporated and the residue is taken up in a mixture of 10 mL of dichloromethane and 10 mL of water. The aqueous phase was extracted with 5 mL of dichloromethane and the combined organic phases are washed successively with a 3 % aqueous solution of hydrochloric acid, saturated aqueous sodium hydrogen carbonate solution, water and brine, dried over magnesium sulphate, filtered and evaporated under reduced pressure. The residue is subjected to flash chromatography on silica gel and then taken directly into the next step without further characterisation.

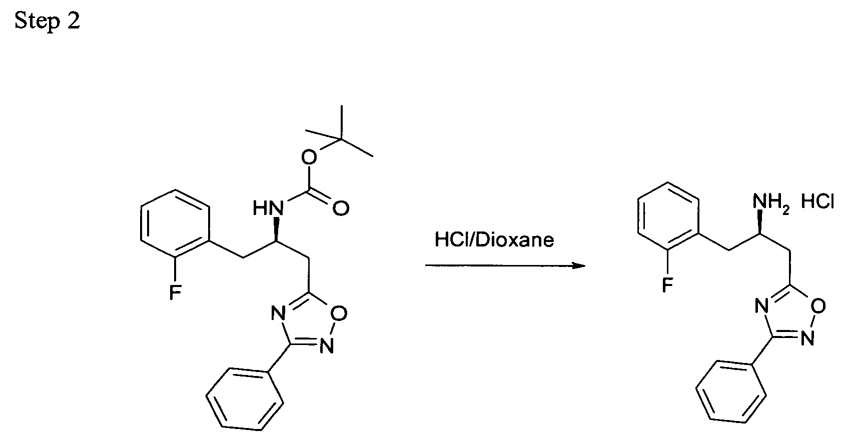

### 2-(2-Fluorophenyl)-(1R)-(3-phenyl-[1,2,4]oxadiazol-5-ylmethyl)-ethylamine. hydrochloride

[2-(2-Fluorophenyl)-(1*R*)-(3-phenyl-[1,2,4]oxadiazol-5-ylmethyl)-ethyl]-carbamic acid *tert*-butyl ester (Example 6, Step 1) is dissolved in 15 mL of dioxane saturated with HCl gas while being stirred and cooled to 5-10°C. The reaction mixture is allowed to warm to room temperature and stirred until the reaction is complete (monitored by TLC: Kieselgel Merck 5554 sheets, eluent: dichloroethane-ethanol 4:1). Evaporation of the solvents under reduced pressure affords a solid residue which is taken up in diethyl ether and n-hexane. The title compound is filtered of, washed with diethylether and dried.
¹H-NMR (300 MHz, CDCl₃ + DMSO-d₆): δ = 3.00-3.48 (4H, m, 2 x CH₂), 4.10 (1H, m, CH), 7.05 (1H, m, fluorophenyl 3-H), 7.11 (1H, m, fluorophenyl 5-H), 7.24 (1H, m, fluorophenyl 4-H), 7.38 (1H, m, fluorophenyl 6-H), 7.48-7.57 (3H, m, phenyl 3,4,5-H), 8.00 (2H, m, phenyl 2,6-H), 8.70 (3H, bs, NH₃⁺).

### Example 7

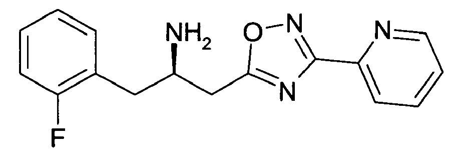

Prepared according to the procedure above outlined for Example 6 Steps 1 to 2, according to Schemes B and F.

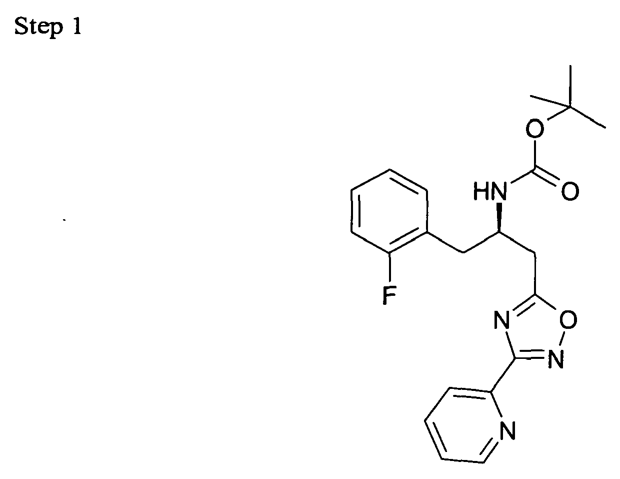

### [2-(2-Fluorophenyl)-(1R)-(3-pyridin-2-yl-[1,2,4]oxadiazol-5-ylmethyl)-ethyl]-carbamic acid tert-butyl ester

Obtained from *N*-hydroxypyridine-2-carboxamide (Example 2) and (3*R*)*-tert-*butoxycarbonylamino-4-(2-fluoro-phenyl)-butyric acid, synthesised according to the procedure for Example 6, Step 1.

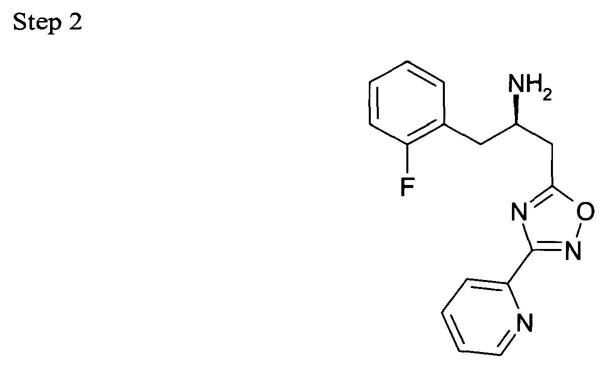

### 2-(2-Fluorophenyl)-(1R)-(3-pyridin-2-yl-[1,2,4]oxadiazol-5-ylmethyl)-ethylamine.

Obtained from [2-(2-Fluorophenyl)-(1*R*)-(3-pyridin-2-yl-[1,2,4]oxadiazol-5-ylmethyl)-ethyl]-carbamic acid *tert*-butyl ester (Example 7, Step 1), and synthesised according to the procedure for Example 6, Step 2 except that after evaporation of the solvents under reduced pressure, the free base is liberated by dissolving the residue in a mixture of 10% aqueous sodium carbonate solution in dichloromethane to afford the title compound as an oil.
¹H-NMR (300 MHz, CDCl₃): δ = 1.60 (2H, broad m, NH₂), 2.80-3.20 (4H, m, 2 x CH₂), 3.76 (1H, m, CH), 7.03-7.27 (4H, m, phenyl), 7.42 (1H, m, pyridine 5-H), 7.82 (1H, m, pyridine 4-H), 8.12 (1H, dd, pyridine 3-H), 8.81 (1H, dd, pyridine 6-H).

### Example 8

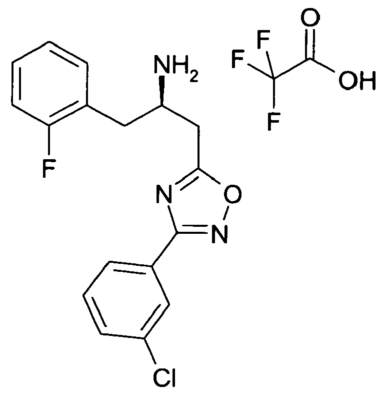

Prepared according to the procedure above outlined for Example 6 Steps 1 to 2, according to Schemes B and F.

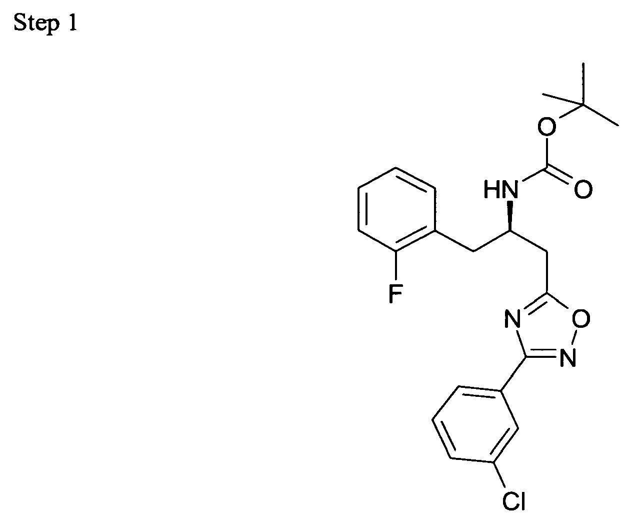

### [(1R)-[3-(3-Chlorophenyl)-[1,2,4]oxadiazol-5-ylmethyl]-2-(2-fluorophenyl)-ethyl]-carbamic acid tert-butyl ester

Obtained from 3-chloro-*N*-hydroxy-benzamidine (Example 3) and (3*R*)*-tert-*butoxycarbonylamino-4-(2-fluoro-phenyl)-butyric acid, synthesised according to the procedure for Example 6, Step 1.

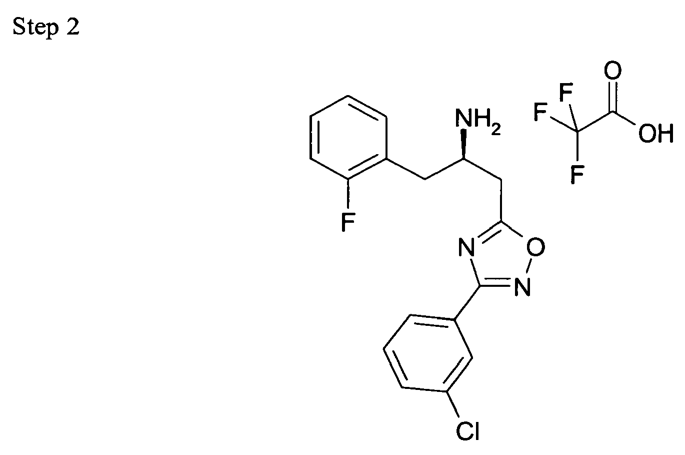

### (1R)-[3-(3-Chlorophenyl)-[1,2,4]oxadiazol-5-ylmethyl]-2-(2-fluorophenyl)-ethylamine. trifluoroacetate

Obtained from [(1*R*)-[3-(3-chlorophenyl)-[1,2,4]oxadiazol-5-ylmethyl]-2-(2-fluorophenyl)-ethyl]-carbamic acid *tert*-butyl ester (Example 8, Step 1), and synthesised according to the procedure for Example 6, Step 2, except that in place of the 15 mL of dioxane saturated with hydrogen chloride gas, a solution of trifluoroacetic acid (ten times excess compare to the compound) in dichloromethane is used.
¹H-NMR (300 MHz, CDCl₃) (partial spectra): δ = 3.00-3.42 (4H, m, 2 x CH₂), 4.10 (1H, m, CH), 7.04 (1H, m, fluorophenyl 3-H), 7.10 (1H, m, fluorophenyl 5-H), 7.20 (1H, m, fluorophenyl 6-H), 7.27 (1H, m, fluorophenyl 4-H), 7.37 (1H, dd, 3-chlorophenyl 5-H), 7.46 (1H, m, 3-chlorophenyl 4-H), 7.89 (1H, m, 3-chlorophenyl 6-H), 7.99 (1H, dd, 3-chlorophenyl 2-H).

### Example 9

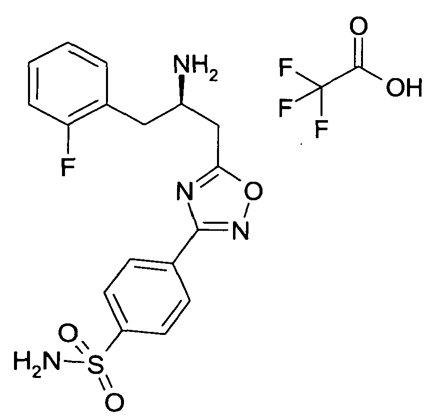

Following example is prepared according to Schemes B and F.

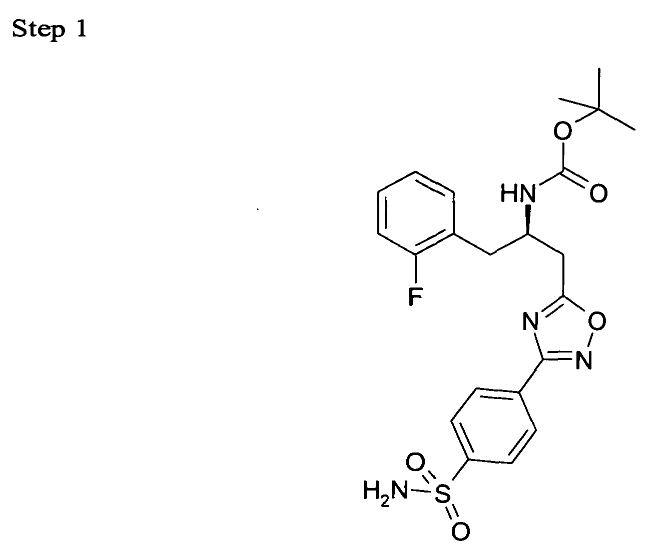

### {2-(2-Fluorophenyl)-(1R)-[3-(4-sulphamoylphenyl)-[1,2,4]oxadiazol-5-ylmethyl]-ethyl}-carbamic acid tert-butyl ester

To a solution of 25.0 mg (0.08 mmol) of (3*R*)-*tert*-butoxycarbonylamino-4-(2-fluorophenyl)-butyric acid in 750 µL *N, N*-dimethylformamide is added 16.4 mg (0.10 mmol) 1,1'-carbonyldiimidazole and the reaction mixture is stirred by room temperature for 1 hour. Then 31.0 mg (0.10 mmol, purity ca. 70%) of *N*-hydroxy-4-sulphamoyl-benzamidine (Example 4) is added and the solution is stirred overnight. After the addition of 2 mL pyridine the solution is refluxed for 1.5 hours, then cooled to room temperature and the solvents are removed under reduced pressure. The crude material is purified using flash chromatography (silica gel, dichloromethane/methanol with 1% ammonia, linear gradient from 0-5% methanol) and it is taken directly into the next step without further characterisation.

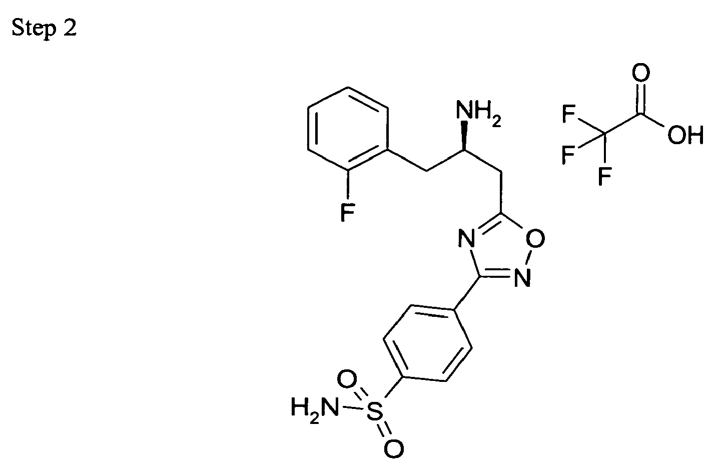

### 4-{5-[(2R)-Amino-3-(2-fluorophenyl)-propyl]-[1,2,4]oxadiazol-3-yl}-benzenesulphonamide. trifluoroacetate

A solution of 15.0 mg (0.03 mmol) of {2-(2-fluorophenyl)-(1*R*)-[3-(4-sulphamoylphenyl)-[1,2,4]oxadiazol-5-ylmethyl]-ethyl}-carbamic acid *tert*-butyl ester (Example 9, Step1) in 1 mL trifluoroacetic acid/dichloromethane (1:2) is stirred for 1 hour by room temperature, then the solvents are removed under reduced pressure and the residue is taken up in methanol and evaporated again to give the title compound.
LC/MS (Method II) rt 1.92 minutes, m/z 377 [M+H]⁺

### Example 10

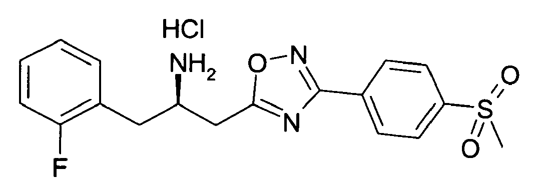

Prepared according to the procedure above outlined for Example 6 Steps 1 to 2, according to Schemes B and F.

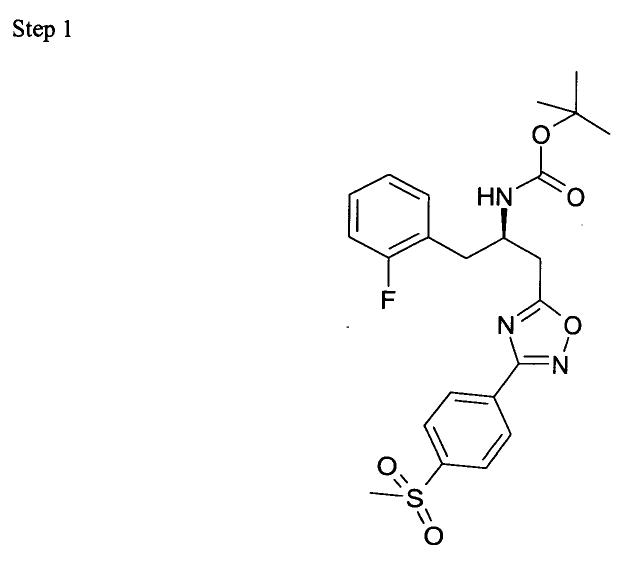

### {2-(2-Fluorophenyl)-(1R)-[3-(4-methanesulphonylphenyl)-[1,2,4]oxadiazol-5-ylmethyl]-ethyl}-carbamic acid tert-butyl ester

Obtained from *N*-hydroxy-4-methanesulphonyl-benzamidine (Example 5) and of (3*R*)-*tert*-butoxycarbonylamino-4-(2-fluoro-phenyl)-butyric acid, synthesised according to the procedure for Example 6, Step 1.

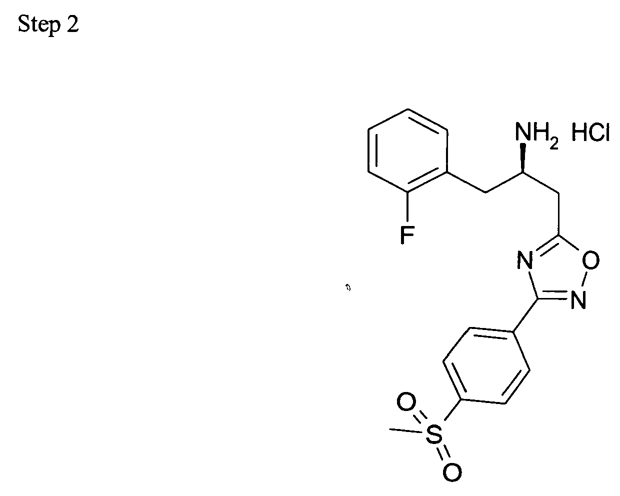

### 2-(2-Fluorophenyl)-(1R)-[3-(4-methanesulphonylphenyl)-[1,2,4]oxadiazol-5-ylmethyl]-ethylamine. hydrochloride

Obtained from {2-(2-Fluorophenyl)-(1*R*)-[3-(4-methanesulphonylphenyl)-[1,2,4]oxadiazol-5-ylmethyl]-ethyl}-carbamic acid *tert-*butyl ester (Example 10, Step 1), and synthesised according to the procedure for Example 6, Step 2.
¹H-NMR (300 MHz, DMSO-d₆): δ = 3.31 (3H, s, CH₃), 3.00-3.60 (4H, m, 2 x CH₂), 4.06 (1H, m, CH), 7.14 (2H, m, fluorophenyl 3,5-H), 7.23 (1H, m, fluorophenyl 4-H), 7.40 (1H, m, 2-fluorophenyl 6-H), 8.12 and 8.21 (4H, m, methylsulfonyl-phenyl 2,3,5,6-H), 8.58 (3H, bs, NH⁺₃).

### ASSAY

Inhibition of DPP-IV peptidase activity was monitored with a continuous fluorimetric assay. This assay is based on the cleavage of the substrate Gly-Pro-AMC (Bachem) by DPP-IV, releasing free AMC. The assay is carried out in 96-well microtiterplates. In a total volume of 100 µl, compounds are preincubated with 50 pM DPP-IV employing a buffer containing 10mM Hepes, 150mM NaCl, 0.005% Tween 20 (pH 7.4). The reaction is started by the addition of 16 µM substrate and the fluorescence of liberated AMC is detected for 10 minutes at 25 °C with a fluorescence reader (BMG-Fluostar; BMG-Technologies) using an excitation wavelength of 370 nm and an emission wavelength of 450 nm. The final concentration of DMSO is 1 %. The inhibitory potential of the compounds were determined. DPP-IV activity assays were carried out with human and porcine DPP-IV (see below); both enzymes showed comparable activities.

Soluble human DPP-IV lacking the transmembrane anchor (Gly31-Pro766) was expressed in a recombinant YEAST-strain as Pre-Pro-alpha-mating fusion. The secreted product (rhuDPP-IV-Gly31-Pro766) was purified from fermentation broth (>90% purity).

The examples 6-10 show a % inhibition of about 2 µM or less.

## Claims

1. A compound of the formula (I)
Z-C(R¹R²)-C(R³NH₂)-C(R⁴R⁵)-A (I)
or a pharmaceutical acceptable salt thereof, wherein
Z is selected from the group consisting of phenyl; naphthyl; indenyl; C₃₋₇ cycloalkyl; indanyl; tetralinyl; decalinyl; heterocycle; and heterobicycle, wherein Z is optionally substituted with one or more R⁶, wherein R⁶ is independently selected from the group consisting of halogen; CN; OH; NH₂; oxo (=O), where the ring is at least partially saturated; R⁷; and R⁸;
R⁷ is selected from the group consisting of C₁₋₆ alkyl; O-C₁₋₆ alkyl; and S-C₁₋₆ alkyl, wherein R⁷ is optionally interrupted by oxygen and wherein R⁷ is optionally substituted with one or more halogen independently selected from the group consisting of F; and Cl;
R⁸ is selected from the group consisting of phenyl; heterocycle; and C₃₋₇ cycloalkyl, wherein R⁸ is optionally substituted with one or more R⁹, wherein R⁹ is independently selected from the group consisting of halogen; CN; OH; NH₂; oxo (=O), where the ring is at least partially saturated; C₁₋₆ alkyl; O-C₁₋₆ alkyl; and S-C₁₋₆ alkyl;
R¹, R⁴ are independently selected from the group consisting of H; F; OH; and R¹⁰;
R², R⁵ are independently selected from the group consisting of H; F; and R¹¹;
R'° is independently selected from the group consisting of C₁₋₆ alkyl; O-C₁₋₆ alkyl; N(R¹²)-C₁₋₆ alkyl; S-C₁₋₆ alkyl; C₃₋₇ cycloalkyl; O-C₃₋₇ cycloalkyl; N(R¹²)-C₃₋₇ cycloalkyl;
S-C₃₋₇ cycloalkyl; -C₁₋₆ alkyl-C₃₋₇ cycloalkyl; O-C₁₋₆ alkyl-C₃₋₇ cycloalkyl; N(R¹²)-C₁₋₆ alkyl-C₃₋₇ cycloalkyl; S-C₁₋₆ alkyl-C₃₋₇ cycloalkyl; heterocycle; O-heterocycle; N(R¹²)-heterocycle; S-heterocycle; C₁₋₆ alkyl-heterocycle; O-C₁₋₆ alkyl-heterocycle; N(R¹²)-C₁₋₆ alkyl-heterocycle; S-C₁₋₆ alkyl-heterocycle; wherein R¹⁰ is optionally substituted with one or more halogen independently selected from the group consisting of F; and Cl;
R¹² is selected from the group consisting of H; and C₁₋₆ alkyl;
R¹¹ is independently selected from the group consisting of C₁₋₆ alkyl; C₃₋₇ cycloalkyl; and -C₁₋₆ alkyl-C₃₋₇ cycloalkyl, wherein R¹¹ is optionally substituted with one or more R¹³, wherein R¹³ is independently selected from the group consisting of F; Cl; and OH;
R³ is selected from the group consisting of H; and C₁₋₆ alkyl;
Optionally one or more pairs of R¹, R², R³, R⁴, R⁵ independently selected from the group consisting of R¹/R²; R²/R³ and R⁴/R⁵; form a C₃₋₇ cycloalkyl ring, which is optionally substituted with one or more of R¹⁴, wherein R¹⁴ is independently selected from the group consisting of F; Cl; and OH;
A is selected from the group consisting of a 5-membered heterocycle having two double bonds and with at least one nitrogen and one additional heteroatom selected from the group consisting of nitrogen; oxygen; and sulphur, as ring atoms; and a 6-membered heterocycle having three double bonds and with at least one nitrogen and one additional heteroatom selected from the group consisting of nitrogen; oxygen; and sulphur, as ring atoms; wherein A is substituted with one or more R¹⁵;
Optionally, A is substituted with one or more R¹⁶, wherein R¹⁶ is independently selected from the group consisting of F; OH; O-C₁₋₆ alkyl; NH₂; NH-C₁₋₆ alkyl; N(C₁₋₆ alkyl)₂; SH; S-C₁₋₆ alkyl; and C₁₋₆ alkyl, wherein each C₁₋₆ alkyl is optionally substituted with one or more halogen selected from the group consisting of F; and Cl;
R¹⁵ is selected from the group consisting of T; -Y-H; and -Y-T;
Y is selected from the group consisting of a covalent bond; -C₁₋₆ alkyl-T⁰-; -O-T⁰-; -O-T⁰-C₁₋₆ alkyl-; -C₁₋₆ alkyl-O-T⁰-; -S-T⁰-; -S-T⁰-C₁₋₆ alkyl-; -C₁₋₆ alkyl-S-T⁰-; -S(O)-T⁰-;
-S(O) -T⁰-C₁₋₆ alkyl-; -S(O)₂-T⁰-; -S(O)₂-T⁰-C₁₋₆ alkyl-; -C₁₋₆ alkyl-S(O)-T⁰-; -C₁₋₆ alkyl-S(O)₂-T⁰-; -N(R¹⁷)-T⁰-; -N(R¹⁷)-T⁰-C₁₋₆ alkyl-; -C₁₋₆ alkyl-N(R¹⁷)-T⁰-; -C(O)-O-; -C(O)O-C₁₋₆ alkyl-T⁰-; -C₁₋₆ alkyl-C(O)O-; -C₁₋₆ alkyl-C(O)O-C₁₋₆ alkyl-T⁰-;-C(O)N(R¹⁷)-; -C(O)N(R¹⁷)-C₁₋₆ alkyl-T⁰-; -C₁₋₆ alkyl-C(O)N(R¹⁷)-; and -C₁₋₆ alkyl-C(O)N(R¹⁷)- C₁₋₆ alkyl-T⁰-; wherein each C₁₋₆ alkyl is optionally substituted with one or more F;
T° is selected from the group consisting of a covalent bond; -C₁₋₆ alkyl-; -C₁₋₆ alkyl-O-; -C₁₋₆ alkyl-N(R¹⁸)-; -C(O)-; -C(O)-C₁₋₆ alkyl-; -C(O)-C₁₋₆ alkyl-O-; -C(O)-C₁₋₆ alkyl-N(R¹⁸)-;
-C(O)O-; -C(O)O-C₁₋₆ alkyl-; -C(O)O-C₁₋₆ alkyl-O-; -C(O)O-C₁₋₆ alkyl-N(R¹⁸)-;
-C(O)N(R¹⁸)-; -C(O)N(R¹⁸)-C₁₋₆ alkyl-; -C(O)N(R¹⁸)-C₁₋₆ alkyl-O-;
-C(O)N(R¹⁸)-C₁₋₆ alkyl-N(R¹⁹)-; -S(O)₂-N(R¹⁸)-; -S(O)₂-N(R¹⁸)-C₁₋₆ alkyl-; -S(O)₂₋N(R¹⁸)-C₁₋₆ alkyl-O-; -S(O)₂-N(R¹⁸)-C₁₋₆ alkyl-N(R¹⁹)-; -S(O)₂-; -S(O)₂-C₁₋₆ alkyl-; - S(O)₂-C₁₋₆ alkyl-O-; and -S(O)₂-C₁₋₆ alkyl-N(R¹⁸)-; wherein each C₁₋₆ alkyl is optionally substituted with one or more F;
R¹⁷, R¹⁸, R¹⁹ are independently selected from the group consisting of H; and C₁₋₆ alkyl;
T is selected from the group consisting of T¹; and T²;
T¹ is selected from the group consisting of phenyl; naphthyl; and indenyl; wherein T¹ is optionally substituted with one or more R²⁰; wherein R²⁰ is independently selected from the group consisting of halogen; CN; R²¹; COOH; OH; C(O)NH₂; S(O)₂NH₂; S(O)NH₂; COOT³; OT³; ST³; C(O)N(R²²)T³; N(R²²)S(O)T³; N(R²²)S(O)₂T³; S(O)₂N(R²²)T³; S(O)N(R²²)T³ and T³;
T² is selected from the group consisting of C₃₋₇ cycloalkyl; indanyl; tetralinyl; decalinyl; heterocycle; and heterobicycle; wherein T² is optionally substituted with one or more R²³, wherein R²³ is independently selected from the group consisting of halogen; CN; R²⁴; OH; oxo (=O), where the ring is at least partially saturated; NH₂; COOH; C(O)NH₂; S(O)₂NH₂; S(O)NH₂; COOT³; OT³; C(O)N(R²²)T³; N(R²²)S(O)T³; N(R²²)S(O)₂T³; S(O)₂N(R²²)T³; S(O)N(R²²)T³; N(R²²)T³; and T³;
Optionally R²³ is C(O)R²², provided that C(O)R²² is bound to a nitrogen, which is a ring atom of a heterocycle or heterobicycle;
R²¹ is selected from the group consisting of C₁₋₆ alkyl; O-C₁₋₆ alkyl; S-C₁₋₆ alkyl; COO-C₁₋₆ alkyl; OC(O)- C₁₋₆ alkyl; C(O)N(R²⁵)-C₁₋₆ alkyl; S(O)₂N(R²⁵)-C₁₋₆ alkyl; S(O)N(R²⁵)-C₁₋₆ alkyl; S(O)-C₁₋₆ alkyl; S(O)₂-C₁₋₆ alkyl; N(R²⁵)S(O)₂-C₁₋₆ alkyl; and N(R²⁵)S(O) -C₁₋₆ alkyl; wherein each C₁₋₆ alkyl is optionally substituted with one more R²⁶, wherein R²⁶ is independently selected from the group consisting of F; COOR²⁷; C(O)N(R²⁷R²⁸); S(O)₂N(R²⁷R²⁸); OR²⁷; N(R²⁷R²⁸); T³; O-T³; and N(R²⁷)-T³;
R²⁴ is selected from the group consisting of C₁₋₆ alkyl; O-C₁₋₆ alkyl; S-C₁₋₆ alkyl; N(R²⁵)-C₁₋₆ alkyl; COO-C₁₋₆ alkyl; OC(O)- C₁₋₆ alkyl; C(O)N(R²⁵)- C₁₋₆ alkyl; N(R²⁵)-C(O)-C₁₋₆ alkyl; S(O)₂N(R²⁵)-C₁₋₆ alkyl; S(O) N(R²⁵)-C₁₋₆ alkyl; S(O)-C₁₋₆ alkyl; S(O)₂-C₁₋₆ alkyl; -N(R²⁵)S(O)₂-C₁₋₆ alkyl; and -N(R²⁵)S(O)-C₁₋₆ alkyl; wherein each C₁₋₆ alkyl is optionally substituted with one or more R^{26a}, wherein R^{26a} is independently selected from the group consisting of F; COOR²⁷; C(O)N(R²⁷R²⁸); S(O)₂N(R²⁷R²⁸); S(O)N(R²⁷R²⁸); OR²⁷; N(R²⁷R²⁸); T³; O-T³; and N(R²⁷)-T³;
R²², R²⁵, R²⁷, R²⁸ are independently selected from the group consisting of H; and C₁₋₆ alkyl;
T³ is selected from the group consisting of T⁴; and T⁵;
T⁴ is selected from the group consisting of phenyl; naphthyl; and indenyl; wherein T⁴ is optionally substituted with one or more R²⁹, wherein R²⁹ is independently selected from the group consisting of halogen; CN; COOR³⁰; OR³⁰; C(O)N(R³⁰R³¹); S(O)₂N(R³⁰R³¹); C₁₋₆ alkyl; O-C₁₋₆ alkyl; S-C₁₋₆ alkyl; COO-C₁₋₆ alkyl; OC(O)-C₁₋₆ alkyl; C(O)N(R³⁰)- C₁₋₆ alkyl; S(O)₂N(R³⁰)-C₁₋₆ alkyl; S(O)N(R³⁰)-C₁₋₆ alkyl; S(O)₂-C₁₋₆ alkyl; S(O)-C₁₋₆ alkyl; N(R³⁰)S(O)₂-C₁₋₆ alkyl; and N(R³⁰)S(O)-C₁₋₆ alkyl; wherein each C₁₋₆ alkyl is optionally substituted with one more halogen selected from the group consisting of F; and Cl;
T⁵ is selected from the group consisting of heterocycle; heterobicycle; C₃₋₇ cycloalkyl; indanyl; tetralinyl; and decalinyl; wherein T⁵ is optionally substituted with one or more R³², wherein R³² is independently selected from the group consisting of halogen; CN; OR³⁰; oxo (=O), where the ring is at least partially saturated;
N(R³⁰R³¹); COOR³⁰; C(O)N(R³⁰R³¹); S(O)₂N(R³⁰R³¹); S(O)N(R³⁰R³¹); C₁₋₆ alkyl; O-C₁₋₆ alkyl; S-C₁₋₆ alkyl; N(R³⁰)-C₁₋₆ alkyl; COO-C₁₋₆ alkyl; OC(O)- C₁₋₆ alkyl; C(O)N(R³⁰)-C₁₋₆ alkyl;
N(R³⁰)-C(O)-C₁₋₆ alkyl; S(O)₂N(R³⁰)-C₁₋₆ alkyl; S(O)N(R³⁰)-C₁₋₆ alkyl; S(O)₂-C₁₋₆ alkyl;
S(O)-C₁₋₆ alkyl; N(R³⁰)S(O)₂-C₁₋₆ alkyl; and N(R³⁰)S(O)-C₁₋₆ alkyl; wherein each
C₁₋₆ alkyl is optionally substituted with one more halogen selected from the group consisting of F; and Cl;
Optionally R³² is C(O)R³⁰, provided that C(O)R³⁰ is bound to a nitrogen, which is a ring atom of a heterocycle or heterobicycle;
R³⁰, R³¹ are independently selected from the group consisting of H; C₁₋₆ alkyl; C₃₋₇ cycloalkyl; and -C₁₋₆ alkyl-C₃₋₇ cycloalkyl.

2. A compound according to claim 1 of formula (1a) 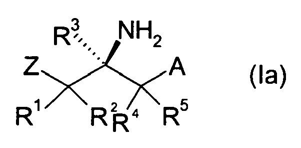
or a pharmaceutically acceptable salt thereof, wherein Z, R¹-R⁵ and A have the meaning as indicated in claim 1.

3. A compound according to claim 1 or 2, wherein Z is selected from the group consisting of phenyl; and heterocycle; and wherein Z is optionally substituted with up to three R⁶, which are the same or different.

4. A compound according to any one of the preceding claims, wherein R⁶ is selected from the group consisting of F; Cl; CN; and C₁₋₆ alkyl.

5. A compound according to any one of the preceding claims, wherein R¹, R², R⁴, R⁵ are independently selected from the group consisting of H; F; and C₁₋₆ alkyl, optionally substituted with one or more F.

6. A compound according to any one of the preceding claims, wherein R³ is H.

7. A compound according to any of the preceding claims, wherein A is selected from the group consisting of oxadiazole; thiadiazole; and triazole.
A compound according to any of the preceding claims, wherein A is substituted with one R¹⁵.

8. A compound according to any of the preceding claims, wherein Y is a covalent bond.

9. A compound according to any of the preceding claims, wherein R¹⁵ is phenyl, optionally substituted with up to three R²⁰, which are the same or different.

10. A compound according to any of the preceding claims, wherein R²⁰ is selected from the group consisting of CH₃; CH₂F; CHF₂; CF₃; CH₂CF₃; F; Cl; S(O)₂NH₂; and CH₃S(O)₂.

11. A compound according to any of the preceding claims, wherein R¹⁵ is a heterocycle.

12. A compound according to claim 11, wherein R¹⁵ is pyridine.

13. A compound according to claim 1 selected from the group consisting of

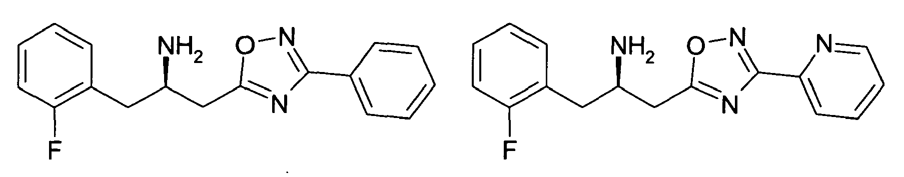

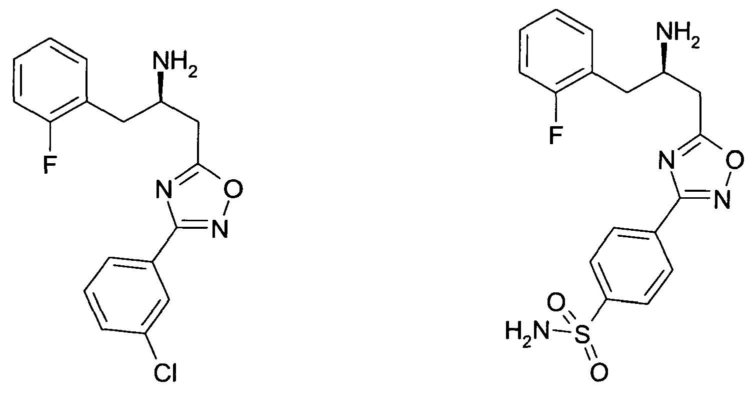

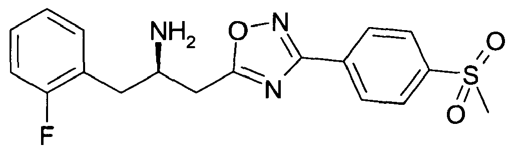

14. A prodrug compound of a compound according to any one of the claims 1 to 13.

15. A pharmaceutical composition comprising a compound or a pharmaceutically acceptable salt thereof according to any one of the claims 1 to 14 together with a pharmaceutically acceptable carrier.

16. A pharmaceutical composition according to claim 15, comprising one or more additional compounds or pharmaceutically acceptable salts thereof selected from the group consisting of another compound according to any one of the claims 1 to 14; another DPP-IV inhibitor; insulin sensitizers; PPAR agonists; biguanides; protein tyrosinephosphatase-1B (PTP-1B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoIy dual agonists; inhibitors of cholesterol absorption; acyl CoA : cholesterol acyltransferase inhibitors; anti-oxidants; PPARo agonists; antiobesity compounds; an ileal bile acid transporter inhibitor; and anti-inflammatory agents.

17. A compound or a pharmaceutically acceptable salt thereof of any one of the claims 1 to 14 for use as a medicament.

18. Use of a compound or a pharmaceutically acceptable salt thereof of any of the claims 1 to 14 for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HN infection; neutropenia; neuronal disorders; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; retinopathy; nephropathy; neuropathy; Syndrome X; ovarian hyperandrogenism (polycystic ovarian syndrome; Type n diabetes; or growth hormone deficiency.

19. Use of a compound according to any one of the claims 1 to 14 as a DPP-IV inhibitor.
